# EUROPEAN PATENT APPLICATION

(11) **EP 4 268 804 A1**
(43) Date of publication of application: **01.11.2023**
(21) Application number: 21908103.1
(22) Date of filing: 21.12.2021
(51) Int. Cl.: A61K 9/14, A61K 8/34, A61K 8/64, A61K 8/73, A61K 47/10, A61K 47/36, A61K 47/38, A61K 47/42

(54) **FIBROIN MICRO-SPHERE AND METHOD FOR PRODUCING SAME**

(30) Priority: 24.12.2020 JP 2020215425
(71) Applicant: University of Tsukuba, Ibaraki 305-8577 (JP)
(72) Inventor: YAMAMOTO Yohei, Tsukuba-shi, Ibaraki 305-8577 (JP); HEAH, Wey Yih, Tsukuba-shi, Ibaraki 305-8577 (JP); YAMAGISHI Hiroshi, Tsukuba-shi, Ibaraki 305-8577 (JP)
(74) Representative: J A Kemp LLP
(86) International application number: PCT/JP2021/047324
(87) International publication number: WO 2022/138640

(57) **Abstract**

The present invention provides a method for producing natural polymer microspheres, the method including: adding a solution containing a natural polymer (e.g., an aqueous fibroin solution) to an organic solvent, the organic solvent having a surfactant dissolved therein, and homogenizing the resulting mixed solution to generate a natural polymer colloidal suspension; and adding the natural polymer colloidal suspension to an alcohol having 3 or more carbon atoms to form natural polymer microspheres.

## Description

### Technical Field

The present invention relates to microspheres of a natural polymer such as silk fibroin and a method for producing the same. The present invention also relates to use of such natural polymer microspheres, particularly fibroin microspheres.

### Background Art

Microspheres are made from a biodegradable material. For example, there is a report on microspheres including silk protein, which is a natural polymer protein (Non-Patent Documents 1 to 3 and the like). However, as disclosed in Non-Patent Documents 1 and 3, the microspheres have a large size and/or a non-uniform shape, and application to cosmetics or the like is difficult for such a reason that the usage sensation is impaired. In Non-Patent Document 2, the microspheres are made from a material as a composite with polyvinyl alcohol (PVA).

In particular, fibroin has attracted attention for use as a drug delivery system, and its drug release rate, release time, drug encapsulation ability, and the like have been addressed (for example, Patent Document 1), but optical properties of fibroin have not been focused on. Accordingly, the surface morphology of a fibroin material is generally rough and the form thereof is irregular.

### Citation List

### Patent Document

Patent Document 1: JP 2015-512944 T

### Non-Patent Document

Non-Patent Document 1: Myung, SJ et al., Macromolecular Research, Vol. 16, pp. 604-608, 2008.
Non-Patent Document 2: Wang, X et al., Biomaterials, Vol. 31(6), pp. 1025-1035, 2010.
Non-Patent Document 3: Oh, H et al., Fibers and Polymers, Vol, 8, pp. 470-476, 2007.

### Summary of Invention

### Technical Problem

There is no report on the production of natural polymer microspheres, particularly silk microspheres having a particle diameter on a micrometer scale (about 2 to 20 µm) and a smooth surface shape. Furthermore, for safety and environmental load reduction, a probability of a method for producing 100% natural microspheres has been desired.

### Solution to Problem

As a result of studying for solving the issue, the present inventors have found that in a case where an alcohol such as isopropanol or n-butanol is used as an alcohol in the production of fibroin microspheres by a reverse miniemulsion method and recrystallization in the alcohol, microspheres having a uniform particle diameter on a micrometer scale, a smooth surface, and a shape very close to a true sphere can be obtained, thereby completing the present invention.

For example, the present invention includes the following embodiments:
[1] A method for producing natural polymer microspheres, the method including:
   adding a solution containing a natural polymer to an organic solvent, the organic solvent having a surfactant dissolved therein, and homogenizing a resulting mixed solution to generate a natural polymer colloidal suspension; and
   adding the natural polymer colloidal suspension to an alcohol having 3 to 4 carbon atoms to form natural polymer microspheres.
[2] The method according to [1], in which the alcohol includes at least one selected from the group consisting of 1-propanol, isopropanol, n-butanol, isobutanol, 2-butanol, and t-butanol.
[3] The method according to [1] or [2], in which the alcohol includes isopropanol or n-butanol.
[4] The method according to any one of [1] to [3], in which the natural polymer includes fibroin, chitosan, cellulose, cotton, wool, or a polysaccharide.
[5] The method according to [4], in which the fibroin is derived from an insect belonging to Bombyx or Antheraea.
[6] The method according to [4] or [5], in which the fibroin is derived from Bombyx mori.
[7] The method according to any one of [1] to [6], in which the solution is an aqueous solution.
[8] The method according to any one of [1] to [7], in which the surfactant includes a nonionic surfactant.
[9] The method according to [8], in which the nonionic surfactant includes at least one selected from the group consisting of a sorbitan fatty acid ester, a glycerin fatty acid ester, and TRITON X-100.
[10] The method according to any one of [1] to [9], in which the organic solvent includes at least one selected from the group consisting of n-hexane, cyclohexane, pentane, heptane, octane, toluene, and xylene.
[11] The method according to any one of [1] to [10], further including adding a fluorescent dye to a suspension of the formed natural polymer microspheres to produce natural polymer microspheres containing the fluorescent dye.
[12] Fibroin microspheres having the following properties:
   (a) the microspheres having a particle diameter of 0.2 to 100 µm; and
   (b) the microspheres having a smooth surface.
[13] The fibroin microspheres according to [12], in which at least 90% of the microspheres have a particle diameter of 0.5 to 20 µm.
[14] The fibroin microspheres according to [12] or [13], in which the microspheres generates whispering gallery mode (WGM) oscillation upon irradiated with excitation light.
[15] The fibroin microspheres according to any one of [12] to [14], further having the following property:
   (c) the microspheres having a uniform size.
[16] The fibroin microspheres according to any one of [12] to [15], further having the following property:
   (d) the microspheres having a spherical shape close to a true sphere.
[17] The fibroin microspheres according to any one of [12] to [16], including a fluorescent dye.
[18] Natural polymer microspheres produced by the method described in any one of [1] to [11].
[19] A composition including the fibroin microspheres described in any one of [12] to [18].
[20] The composition according to [19], which is a cosmetic composition.
[21] A composition including the fibroin microspheres described in any one of [12] to [18], which is for use in cosmetics.
[22] A cosmetic method for a subject, the cosmetic method including applying or topically applying a composition including the fibroin microspheres described in any one of [12] to [18] to a skin of the subject.

### Advantageous Effects of Invention

According to the present invention, it is possible to obtain natural polymer microspheres, particularly fibroin microspheres having a small size on a micrometer scale and an extremely smooth surface. In addition, such natural polymer microspheres, particularly the fibroin microspheres, are extremely close to a true sphere in shape, smooth in surface, and uniform in size, and thus can be expected to be used as a novel cosmetic material, for example. Furthermore, the natural polymer microspheres according to the present invention are biodegradable, so that an environmental load is low. Accordingly, the present invention can be used in fields of cosmetic materials, cosmetic development, cosmetic production, food and beverage, and the like.

### Brief Description of Drawings

FIG. 1 is a flowchart of an example of a method for producing natural polymer (e.g., fibroin) microspheres.
FIG. 2 indicates scanning electron microscope (SEM) images of fibroin microspheres. The following alcohols were used for resolubilization in preparing microspheres. A: isopropanol, B: n-butanol, C: 1: 1 liquid mixture of methanol and ethanol, D: ethanol.
FIG. 3 indicates a microscopic image of fibroin microspheres after staining with Acid Red 52.
FIG. 4 indicates microfluorescence spectroscopy (µ-PL) spectra of fibroin microspheres. The following alcohols were used for resolubilization in preparing microspheres. A: isopropanol, B: n-butanol, C: 1: 1 liquid mixture of methanol and ethanol, D: ethanol.
FIG. 5 is a graph indicating a particle diameter distribution of fibroin microspheres.
FIG. 6 is a graph indicating a change in weight of fibroin microspheres caused by proteinase K.

### Description of Embodiments

The present invention claims priority from the Japanese Patent Application No. 2020-215425 filed on December 24, 2020, the entire contents of which are incorporated herein by reference.

Hereinafter, the present invention will be described in detail.

The present invention relates to microspheres of a natural polymer and a method for producing the same. In the present invention, the "natural polymer" is not particularly limited as long as it is a natural polymer that is desired to be prepared as microspheres. Examples thereof include proteins (fiber proteins) serving as materials of animal fibers such as silk, wool, silk thread, cashmere, and angora, and specific examples thereof include fibroin, keratin, collagen, and elastin. Further examples thereof include chitosan, cellulose, cotton, wool, and polysaccharides (such as starch). The word "natural" may encompass a polymer subjected to pretreatment such as purification or extraction, or a polymer recombinantly produced on the basis of its sequence information, as long as the polymer is originally derived from a natural organism or environment. It may also encompass a variant or homologue thereof as long as the variant or homologue has a function equivalent to that of a naturallyderived polymer. For example, a polymer in which one or several amino acids are deleted, substituted, or added in an amino acid sequence of a naturally-occurring polymer (protein) and which retains the function or activity of the original polymer is also included in the "natural polymer" in the present invention.

In the present invention, fibroin is particularly targeted as the natural polymer. Fibroin is a protein that is a main component of silk (silken thread) and is used not only in textile products but also in cosmetics, foodstuffs, and pharmaceuticals. In the present invention, such fibrous fibroin is prepared and utilized in a form of spheres (microspheres) having a micrometer-scale particle diameter.

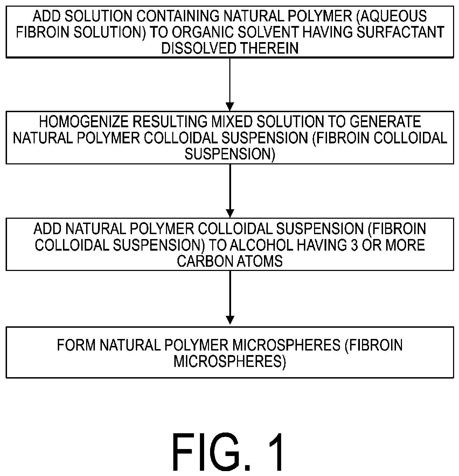

A method for producing natural polymer microspheres mainly includes performing a reverse miniemulsion method and recrystallization in an alcohol. FIG. 1 indicates a flowchart of an example of the method for producing natural polymer (e.g., fibroin) microspheres according to the present invention. For example, when fibroin is used as the natural polymer, fibroin, which is an insoluble polymer, is dissolved and again converted into a β-sheet to form uniform and fine spherical bodies. In the present invention, an alcohol having 3 or more carbon atoms is used in place of methanol or ethanol used as the alcohol in the related art in the recrystallization.

Specifically, the method for producing natural polymer (such as fibroin) microspheres according to the present invention includes:
a reverse miniemulsion method: adding a solution containing a natural polymer (for example, an aqueous fibroin solution) to an organic solvent in which a surfactant is dissolved and homogenizing the resulting mixed solution to generate a natural polymer colloidal suspension; and
recrystallization in alcohol: adding the natural polymer colloidal suspension to an alcohol having 3 or more carbon atoms to form natural polymer microspheres.

The natural polymer can be usually prepared by those skilled in the art depending on the purpose, or a commercially available product can be purchased.

The solution in which the natural polymer is dissolved can be appropriately selected by those skilled in the art depending on a type and an amount of the natural polymer to be used and types of the surfactant and the organic solvent to be used. For example, the solution may be water, an organic solvent including an alcohol, or the like.

Fibroin is not particularly limited as long as it is fibroin known as silk fibroin, and may be natural fibroin or recombinant fibroin. Fibroin is well known in the art, and any fibroin can be selected by those skilled in the art. Amino acid sequence information of the fibroin protein is also known, and recombinant fibroin can be prepared by a method known in the art.

In the present invention, any fibroin derived from an arthropod having an ability to generate thread (silken thread) can be used as the natural fibroin. Examples of such an arthropod include, but are not limited to:
Lepidoptera,
   Bombycidae,
      Bombyx: Bombyx mori; Bombyx mandarina,
   Saturniidae,
      Antheraea: Antheraea yamamai; Antheraea pernyi,
Araneae,
   Nephilidae,
   Nephila: Nephila clavata; Nephila clavipes; and
Hymenoptera.

Preferably, fibroin derived from an insect belonging to Bombyx or Antheraea, more preferably from Bombyx mori, is used.

A method for preparing the aqueous fibroin solution is also well known in the art. For example, in a case of natural fibroin, the aqueous fibroin solution can be prepared by removing sericin from cocoons or spider threads of an arthropod as described above by scouring, dissolving the resulting fibroin in an aqueous salt solution, and then desalting the solution.

The scouring can be performed by any method known in the art. For example, cocoons or spider threads are boiled in an aqueous solution for about 30 minutes to about 3 hours at about 97°C to a boiling temperature. The aqueous solution used for the scouring may contain an alkaline agent or a proteolytic enzyme, and for example, a weakly alkaline scouring agent such as soap, sodium carbonate (soda ash), sodium bicarbonate (baking soda), or sodium silicate (water glass) is used in the related art. In a preferred embodiment, cocoons of Bombyx mori are boiled in an aqueous solution of 0.02 M sodium carbonate (NaCO₃) for about 30 minutes to remove sericin. Alternatively, fibroin after scouring may be purchased and used.

The fibroin after removing sericin is dissolved in an aqueous salt solution containing a reagent capable of dissolving fibroin (e.g., lithium bromide, lithium thiocyanate, calcium oxalate, or the like). Thereafter, desalting (removal of salts contained in the aqueous salt solution) is performed, for example, by dialysis. In a preferred embodiment, fibroin is dissolved in an about 8 to 12 M lithium bromide (LiBr) solution with stirring, and then dialysis is performed. The obtained aqueous fibroin solution can be adjusted to an appropriate concentration (for example, 1 to 100 g/L).

Subsequently, the reverse miniemulsion method is performed using a solution containing a natural polymer (for example, an aqueous fibroin solution) to form a reverse micelle. First, the solution containing a natural polymer (for example, an aqueous fibroin solution) is added to an organic solvent in which a surfactant is dissolved. The organic solvent to be used is preferably an organic solvent having a low polarity, and for example, n-hexane, cyclohexane, pentane, heptane, octane, toluene, or xylene, or a mixture thereof can be used. The surfactant is preferably a nonionic surfactant, and a sorbitan fatty acid ester such as sorbitan monooleate, sorbitan monostearate, or sorbitan tristearate; a glycerin fatty acid ester, acetic acid monoglyceride, citric acid monoglyceride, diacetyltartaric acid monoglyceride, or the like, TRITON X-100 (octylphenoxypolyethoxyethanol), or the like can be used. In one embodiment, in about 0.5 to 2 mL of the organic solvent, for example, about 30 to 436 mg of sorbitan monooleate is dissolved as the surfactant, and a solution containing a natural polymer, for example, an aqueous fibroin solution (in 3 mg/100 µL) is added thereto. The resulting mixed solution is then homogenized to generate a natural polymer colloidal suspension (e.g., a fibroin colloidal suspension). The homogenization conditions can be appropriately set depending on a type and an amount of the solvent to be used, an amount of the natural polymer, and the like. For example, homogenization is performed at ambient temperature for 2 to 5 minutes at 10 to 30 krpm (e.g., 25 krpm).

Subsequently, recrystallization in an alcohol is performed. Specifically, the obtained natural polymer colloidal suspension is added to an alcohol having 3 or more carbon atoms to form natural polymer microspheres. In a specific embodiment, a natural polymer colloidal suspension obtained from a solution containing about 100 µl of the natural polymer is added to about 2.5 to 20 ml of the alcohol and allowed to stand for about 1 to 24 hours. In an exemplary embodiment, a fibroin colloidal suspension obtained from about 100 µl of an aqueous fibroin solution is added to about 2.5 to 20 ml of the alcohol and allowed to stand for about 1 to 24 hours.

In the present invention, as the alcohol used for recrystallization, an alcohol having 3 or more carbon atoms, for example, an alcohol having 3 to 6 carbon atoms, or preferably an alcohol having 3 to 4 carbon atoms is used. For example, at least one selected from the group consisting of the following is included as the alcohol:
propanol, such as 1-propanol and isopropanol (also referred to as 2-propanol);
butanol, such as n-butanol (also referred to as 1-butanol), isobutanol (also referred to as 2-methyl-1-propanol), 2-butanol (also referred to as s-butyl alcohol), and t-butanol (also referred to as 2-methyl-2-propanol);
pentanol, such as 1-pentanol, 2-pentanol, 3-pentanol, 2-methyl-1-butanol, 2-methyl-2-butanol, 3-methyl-1-butanol, 3-methyl-2-butanol, and 2,2-dimethyl-1-propanol; and
hexanol, such as 1-hexanol, 2-hexanol, 3-hexanol, 2-methyl-1-pentanol, 3-methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, 2,2-dimethyl-1-butanol, 2,3-dimethyl-1-butanol, 3,3-dimethyl-1-butanol, 2,3-dimethyl-2-butanol, 3,3-dimethyl-2-butanol, and 2-ethyl-1-butanol.

These alcohols may be optionally substituted with an alkyl group or the like.

Preferably, the alcohol used for recrystallization is isopropanol or n-butanol.

This results in natural polymer microspheres in the solution. The formed natural polymer microspheres are collected by centrifugation, and then washed with the used alcohol or the like to remove unnecessary reagents.

The method for producing natural polymer microspheres (e.g., fibroin microspheres) according to the present invention may further include
adding a fluorescent dye to the formed suspension of natural polymer microspheres to generate natural polymer microspheres containing the fluorescent dye. The fluorescent dye is not particularly limited as long as it can exhibit light confinement and light emission enhancement effects by the microspheres. For example, Rhodamine 6G, Rhodamine B, Acid Red 52 (AR-52), or the like can be used. The reaction conditions can be appropriately set by those skilled in the art depending on a type of the fluorescent dye to be used.

The obtained natural polymer microspheres, for example, fibroin microspheres have a particle diameter on a micrometer scale, a smooth surface, a uniform size (particle diameter), and a spherical or substantially spherical shape close to a perfect sphere.

In one aspect, the present invention provides fibroin microspheres, and the fibroin microspheres have at least the following properties:
(a) the microspheres having a particle diameter of 0.2 to 100 µm, preferably a particle diameter of 0.5 to 20 µm, more preferably a particle diameter of 1 to 10 µm, and
(b) the microspheres having a smooth surface.

The particle diameter of the natural polymer microspheres, for example, the fibroin microspheres, can be measured by a known method, for example, observation under a microscope (optical microscope or electron microscope), dynamic light scattering (for example, photon correlation spectroscopy, laser diffraction, laser light scattering, or the like), or the like. Note that the term "particle diameter" or "size" as used herein refers to a particle diameter or size of a single microsphere or an average of particle diameters or sizes of a plurality of microspheres.

In a preferred embodiment, at least 90% of the fibroin microspheres according to the present invention have a particle diameter of 0.2 to 100 µm, preferably a particle diameter of 0.5 to 20 µm, and more preferably a particle diameter of 1 to 10 µm.

It can also be evaluated by a known method that the natural polymer microspheres, for example, the fibroin microspheres have a smooth surface. For example, observation under a microscope (an optical microscope or an electron microscope), evaluation of whispering gallery mode (WGM) oscillation by irradiation with excitation light, or the like can be used. WGM oscillation occurs when a spherical particle is irradiated with excitation light such as laser light, light generated inside the particle is totally reflected in the vicinity of the interface due to a difference in refractive index from the outside (air) and is confined in the spherical particle, and when an integral multiple of a wavelength of the light confined in the particle coincides with an optical path length, a light emission intensity increases and appears as a peak. This WGM oscillation is an indication that the particle has a spherical shape and a smooth surface. Evaluation of WGM oscillation can also be easily performed by those skilled in the art (see, for example, JP 2015-137312 A). In a preferred embodiment, the natural polymer microspheres according to the present invention, for example, the fibroin microspheres, generate whispering gallery mode (WGM) oscillation upon irradiated with excitation light. Note that when the WGM oscillation is evaluated, natural polymer microspheres containing a fluorescent dye may be prepared in advance, or natural polymer microspheres may be produced and then a fluorescent dye may be adsorbed on the surfaces thereof.

The natural polymer microspheres according to the present invention, such as the fibroin microspheres, have preferably a uniform size. For example, the particle diameter or size of the microspheres is within a range of ±1 to 10 microns, preferably within a range of ±1 to 5 microns from the average value or median value thereof.

Preferably, the natural polymer microspheres according to the present invention, for example, the fibroin microspheres have a spherical or substantially spherical shape close to a true sphere. For example, at least 90% of the microspheres have a spherical shape and do not have a squashed spherical or flattened shape. This can be confirmed, for example, by observation under an electron microscope.

The natural polymer microspheres according to the present invention, for example, the fibroin microspheres, are biodegradable because the raw material is a natural polymer. Accordingly, the natural polymer microspheres have a low environmental load and are a preferable material for industrial use.

The natural polymer microspheres according to the present invention, for example, the fibroin microsphere, may contain a fluorescent dye. As described above, the fluorescent dye is not particularly limited as long as it can exhibit the light confinement and light emission enhancement effect by the microspheres, and for example, Rhodamine 6G, Rhodamine B, Acid Red 52 (AR-52), or the like can be used.

The natural polymer microspheres according to the present invention, for example, the fibroin microspheres, can be used as a composition. For example, the natural polymer microspheres (as an example, the fibroin microspheres) can be mixed with a suitable carrier, or diluted or suspended in a carrier to form a composition. Examples of the suitable carrier include salt solutions (such as physiological saline), lactose, dextrose, sucrose, sorbitol, mannitol, xylitol, erythritol, maltitol, starch, gum acacia, alginates, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In addition, the composition may contain an excipient, a surfactant, a dispersant, a buffer, a preservative, a solubilizing agent, a stabilizer, an isotonic agents, or the like, which is commonly used.

The composition containing the fibroin microspheres according to the present invention is considered to have the following effects and uses:
(1) a retroreflective effect (an effect of enhancing skin radiance) by addition to cosmetics;
(2) a moisturizing effect by addition to cosmetics;
(3) a light confinement and light emission enhancement effect (an enhancement effect of skin gross and whitening) by using the fibroin microspheres containing a fluorescent dye; and
(4) a protective effect against ultraviolet rays by addition to cosmetics.

Accordingly, in a preferred embodiment, the natural polymer microspheres, for example, the fibroin microspheres, are blended into cosmetics and used as a cosmetic composition. Examples of the cosmetics into which the natural polymer microspheres are blended include a skin lotion, a milky lotion, a cream, a foundation, a hair care product (a shampoo, a treatment, and the like), a lipstick, a blusher, and an eye shadow. The cosmetic composition may contain a cosmetically acceptable excipient or carrier, such as an extender, a binder, a wetting agent, a lubricant, a surfactant, a dispersant, a buffer, a pH adjusting agent, a preservative, a solubilizing agent, an antiseptic agent, an absorption enhancer, a stabilizer, an isotonic agent, or the like. Depending on its form, the cosmetic composition can be produced by a common method such as mixing, coating, spraying, or the like.

The natural polymer, for example, fibroin, used in the present invention is a natural material, and thus, it can be used for human and animal foods. Accordingly, the natural polymer microspheres, for example, the fibroin microspheres, can be blended into a food or a food additive to be used as a food coating agent, a health food, or the like. The food composition may include an additive that is acceptable as a food. Blending into the food and food additive can be performed by a method such as mixing, dipping, coating, or spraying.

A subject for which the composition according to the present invention is used is not particularly limited. For example, the composition can be applied to or ingested by mammals such as humans, primates (such as monkeys and chimpanzees), livestock animals (such as cows, horses, and pigs), companion animals (such as dogs and cats), or laboratory animals (such as mice, rats, and monkeys). In particular, a human subject who use cosmetics is preferred as the subject.

### Examples

Hereinafter, the present invention will be described in more detail with reference to examples and the drawings. However, the present invention is not limited to the following examples.

### Example 1: Preparation of fibroin microspheres - recrystallization using isopropanol

In the present example, microspheres of silk fibroin (SF) as a natural polymer were prepared by recrystallization using isopropanol (IPA) as an alcohol.

### (1) Scouring of cocoons (removal of sericin)

Cocoons of Bombyx mori were boiled in an aqueous solution of 0.02 M sodium carbonate (Na₂CO₃) for 30 minutes to remove silk sericin, and were washed with ultrapure water three times to remove the residue. The extracted cocoons consisting only of silk fibroin were air-dried at room temperature for 24 hours. The dried silk fibroin was stored in a cool and dark place until use.

### (2) Redissolution of silk fibroin

While 200 mg of the fibroin from which sericin was removed was stirred at 100 rpm for 3 hours at 90°C, 0.8 mL of 9.3 M LiBr solution was dissolved therein. Next, the solution in which the fibroin was dissolved was dialyzed against ultrapure water for 3 days using a dialysis tube (MWCO 12000 to 14000, Kenis) and desalted (LiBr was removed). For the desalting, water was changed 5 times at intervals of 1 hour, 4 hours, 8 hours, 12 hours, and 24 hours. The desalted fibroin solution was centrifuged for 10 minutes to remove silk aggregates and other residues. The final concentration of the aqueous silk fibroin solution was found to be about 3% (w/v) by a dry weight of the solution (3 mg silk fibroin per 100 µL). The calculation method was that, after vacuum drying for one hour at -0.1 MPa and 50°C, a weight of the remaining solid content was measured. The silk fibroin aqueous stock solution was stored at 4°C.

### (3) Preparation of fibroin microspheres

### Reverse miniemulsion method:

Span 80 (sorbitan monooleate, 30 to 436 mg) as a surfactant was added to 0.5 to 2 mL of n-hexane, and the mixture was vigorously shaken to dissolve Span 80 in n-hexane. Next, 100 µL of the fibroin aqueous stock solution (3 mg of silk fibroin) was added into n-hexane using a pipette. The mixture was homogenized with a homogenizer for 2 to 5 minutes at a rotational speed of 10 to 30 krpm to form a fibroin colloidal suspension.

### Recrystallization in alcohol:

Into a 50 mL vial, 2.5 to 20 mL of isopropanol (IPA) and 1 mL of n-hexane were poured in layers, and the fibroin colloidal suspension (100 µL of the aqueous solution) was poured into the uppermost layer using a pipette so as not to disturb the liquid surface. The vial was allowed to stand for 1 to 24 hours to slowly form microspheres in the solution. The fibroin microspheres formed in the solution were collected by centrifugation and washed twice with IPA (each 1.5 ml) and once with ethanol (1.5 ml) to remove the surfactant and n-hexane. The fibroin microspheres were stored in IPA at room temperature.

### Example 2: Preparation of fibroin microspheres - recrystallization using n-butanol

In the present example, microspheres of silk fibroin (SF) were prepared by recrystallization using n-butanol (BuOH) as the alcohol. Specifically, silk fibroin microspheres were prepared by the same procedure as in Example 1 using n-butanol instead of isopropanol.

### Comparative Example

In the present example, microspheres of silk fibroin (SF) were prepared by recrystallization using methanol or ethanol as the alcohol. Specifically, silk fibroin microspheres were prepared by the same procedure as in Example 1 using a 1:1 liquid mixture of methanol and ethanol (MeOH/EtOH) or ethanol (EtOH) instead of isopropanol.

### Example 3: Properties of fibroin microspheres

### (1) Shape and size of microspheres

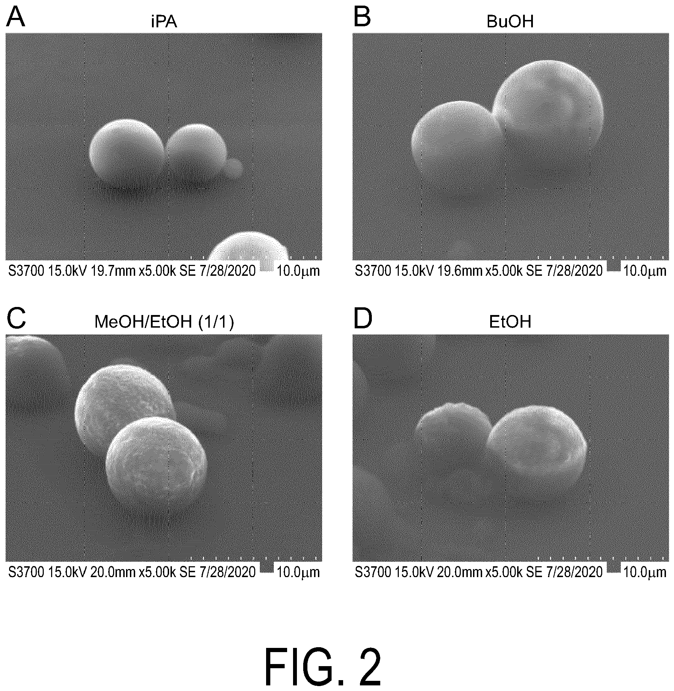

The fibroin microspheres obtained in Examples 1 and 2 and Comparative Example were observed using a scanning electron microscope (SEM). The results are indicated in FIG. 2.

As indicated in A and B of FIG. 2, it was found that the fibroin microspheres (Examples 1 and 2) obtained by using isopropanol (IPA) or n-butanol (BuOH) at the time of recrystallization had a shape close to a true sphere, and also had an extremely smooth surface. In addition, it was found that sizes (particle diameters) of the microspheres obtained in Examples 1 and 2 were about 5 to 6 µm and about 7 to 9 µm, respectively (A and B of FIG. 2). Meanwhile, as indicated in C and D of FIG. 2, the silk microspheres of Comparative Example had rough surfaces, and some of them did not maintain a spherical shape.

### (2) Shape and surface of microspheres

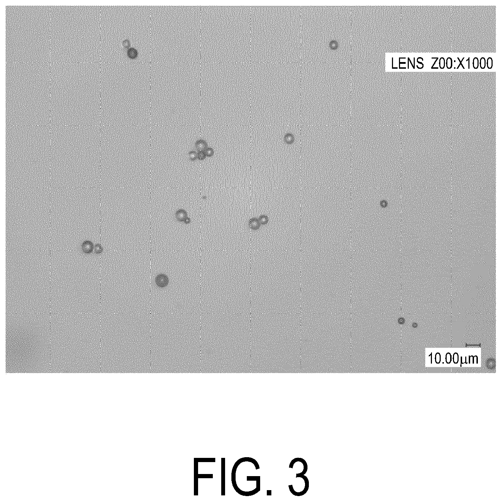

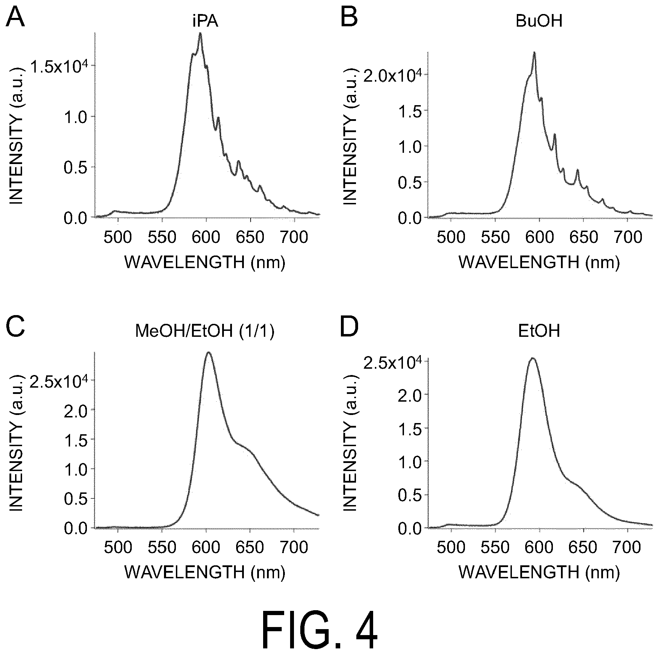

Microfluorescence spectroscopy (µ-PL) of the microspheres was performed to investigate details of a three-dimensional structure of the fibroin microspheres. To impart fluorescent properties to the microspheres, 150 to 300 µg of Acid Red 52 (AR52) or Rhodamine 6G (R6G) in 1 ml of dimethylformamide (DMF) was added to the suspension of fibroin microspheres obtained in Examples 1 and 2 and Comparative Example (1 to 3 mg of fibroin microspheres). The mixture was heated in an oven at 85°C for 6 to 24 hours, then centrifuged, and washed three times with the alcohol used for recrystallization (each 1.5 ml). The resulting fibroin microspheres were examined in a µ-PL system (NIKON industrial microscope, custom use) at 50× and excited with a 450 nm laser, a fluorescence spectrum was captured with a lambda vision LV-MC2 spectrometer, and a fluorescence image was captured with a CCD camera. The results are indicated in FIGS. 3 and 4.

FIG. 3 indicates a microscopic image of the microspheres obtained in Example 1 after staining with Acid Red 52. As indicated in A and B of FIG. 4, the fibroin microspheres obtained in Examples 1 and 2 indicated a whispering gallery mode (WGM) peak at about 600 nm in the µ-PL spectrum. The optical property indicates that the microspheres have a spherical shape and a smooth surface structure. Thus, fluorescence is confined in the microspheres by total reflection at the interface between the fibroin microspheres and air. In contrast, as indicated in C and D of FIG. 4, no WGM peak was observed in the silk microspheres of Comparative Example.

### (3) Uniformity of microspheres

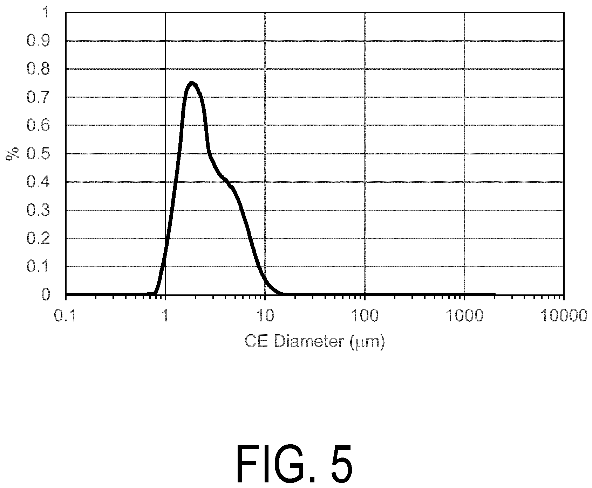

Particle diameter distribution of the fibroin microspheres was evaluated. Specifically, the particle diameter distribution of the microspheres obtained in Example 1 was evaluated using Morphologi 4 (Malvern Panalytical), which is an image analysis-type particle diameter and shape distribution measurement apparatus, in according with its product protocol. The results are indicated in FIG. 5. It can be seen from FIG. 5 that the particle diameters of the microspheres are distributed in a range of about 1 to 8 µm, particularly about 2 to 5 µm, and are uniform.

### (4) Biodegradability of microspheres

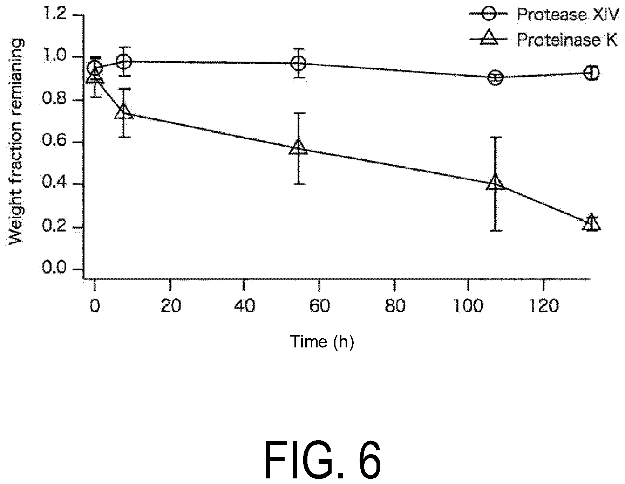

Biodegradability of the fibroin microspheres by proteinase K was evaluated. Specifically, 10 mg of the microspheres obtained in Example 1 and 1 mL of an enzyme solution were prepared and weighed (W₁ mg), and then left to stand with shaking at room temperature for 133 hours. At a plurality of evaluation time points, a supernatant was removed by centrifugation, and the residue was frozen, vacuum-dried at room temperature, and weighed (W₂ mg). Proteinase K was used as the enzyme and protease XIV was used as a control. A size and a wavelength shift of the microspheres were also evaluated. As a result, for a weight change (W₂ - Wi/10 mg), as indicated in FIG. 6, it was found that the weight of the microspheres was decreased over time by proteinase K. Proteinase K also decreased the size of the microspheres over time and a shift in the peak wavelength was also observed. Accordingly, the fibroin microspheres were degraded by proteinase K and were found to be biodegradable, as expected.

All publications, patents, and patent applications cited in the present specification are incorporated herein by reference in their entirety.

## Claims

1. A method for producing natural polymer microspheres, the method comprising:
adding a solution containing a natural polymer to an organic solvent, the organic solvent having a surfactant dissolved therein, and homogenizing a resulting mixed solution to generate a natural polymer colloidal suspension; and
adding the natural polymer colloidal suspension to an alcohol having 3 to 4 carbon atoms to form natural polymer microspheres.

2. The method according to claim 1, wherein the alcohol comprises at least one selected from the group consisting of 1-propanol, isopropanol, n-butanol, isobutanol, 2-butanol, and t-butanol.

3. The method according to claim 1 or 2, wherein the alcohol comprises isopropanol or n-butanol.

4. The method according to any one of claims 1 to 3, wherein the natural polymer comprises fibroin, chitosan, cellulose, cotton, wool, or a polysaccharide.

5. The method according to claim 4, wherein the fibroin is derived from an insect belonging to Bombyx or Antheraea.

6. The method according to claim 4 or 5, wherein the fibroin is derived from Bombyx mori.

7. The method according to any one of claims 1 to 6, wherein the solution is an aqueous solution.

8. The method according to any one of claims 1 to 7, wherein the surfactant comprises a nonionic surfactant.

9. The method according to claim 8, wherein the nonionic surfactant comprises at least one selected from the group consisting of a sorbitan fatty acid ester, a glycerin fatty acid ester, and TRITON X-100.

10. The method according to any one of claims 1 to 9, wherein the organic solvent comprises at least one selected from the group consisting of n-hexane, cyclohexane, pentane, heptane, octane, toluene, and xylene.

11. The method according to any one of claims 1 to 10, further comprising adding a fluorescent dye to a suspension of the formed natural polymer microspheres to generate natural polymer microspheres containing the fluorescent dye.

12. Fibroin microspheres having the following properties:
(a) the microspheres having a particle diameter of 0.2 to 100 µm; and
(b) the microspheres having a smooth surface.

13. The fibroin microspheres according to claim 12, wherein at least 90% of the microspheres have a particle diameter of 0.5 to 20 µm.

14. The fibroin microspheres according to claim 12 or 13, wherein the microspheres generate whispering gallery mode (WGM) oscillation upon irradiated with excitation light.

15. The fibroin microspheres according to any one of claims 12 to 14, further having the following property:
(c) the microspheres having a uniform size.

16. The fibroin microspheres according to any one of claims 12 to 15, further having the following property:
(d) the microspheres having a spherical shape close to a true sphere.

17. The fibroin microspheres according to any one of claims 12 to 16, comprising a fluorescent dye.

18. Natural polymer microspheres produced by the method described in any one of claims 1 to 11.

19. A composition comprising the fibroin microspheres described in any one of claims 12 to 18.

20. The composition according to claim 19, which is a cosmetic composition.
